(19) Europäisches Patentamt
European Patent Office
Office européen des brevets

(11) Veröffentlichungsnummer: **0 679 636 A1**

# (12) EUROPÄISCHE PATENTANMELDUNG

(21) Anmeldenummer: **95110459.5**

(22) Anmeldetag: **17.01.92**

(51) Int. Cl.[6]: **C07C 251/48**, C07C 255/64, C07D 333/22, A01N 35/10, A01N 37/50, A01N 43/10

Diese Anmeldung ist am 05 - 07 - 1995 als Teilanmeldung zu der unter INID-Kode 60 erwähnten Anmeldung eingereicht worden.

(30) Priorität: **07.02.91 DE 4103695**

(43) Veröffentlichungstag der Anmeldung:
**02.11.95 Patentblatt 95/44**

(60) Veröffentlichungsnummer der früheren Anmeldung nach Art. 76 EPÜ: **0 498 188**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**D-67063 Ludwigshafen (DE)**

(72) Erfinder: **Benoit, Remy, Dr.**
**Holzmühlstr. 6**
**D-67435 Neustadt (DE)**
Erfinder: **Sauter, Hubert, Dr.**
**Neckarpromenade 20**
**D-68167 Mannheim (DE)**
Erfinder: **Kirstgen, Reinhard, Dr.**
**Erkenbrechtstr. 23e**
**D-67434 Neustadt (DE)**
Erfinder: **Lorenz, Gisela, Dr.**
**Erlenweg 13**
**D-67434 Hambach (DE)**
Erfinder: **Ammermann, Eberhard, Dr.**
**Von-Gagern-Str. 2**
**D-64646 Heppenheim (DE)**

(54) **Oximether und diese enthaltende Fungizide.**

(57) Oximether der Formel I

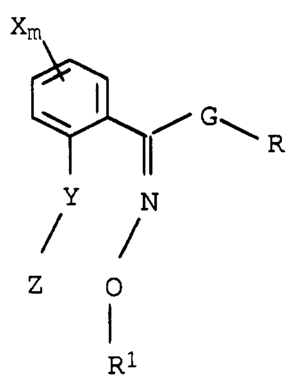

I,

in der
G



Rank Xerox (UK) Business Services
(3.10/3.09/3.3.4)

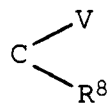

bedeutet,

V  O-$R^6$,

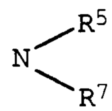

bedeutet,

R  Cycloalkyl, Alkenyl, Alkinyl, Phenyl, Hetaryl oder

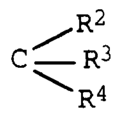

bedeutet,

$R^1$  Wasserstoff, $C_1$-$C_8$-Alkyl bedeutet,

$R^2$  Wasserstoff, Alkyl, Aralkyl, Hetarylalkyl, Alkoxyalkyl, Aryloxyalkyl, Aryl, Hetaryl bedeutet,

$R^3$  Wasserstoff, Methyl, -COO-A bedeutet,

A  Wasserstoff, $C_1$-$C_8$-Alkyl bedeutet, oder

für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$  Wasserstoff, $C_1$-$C_8$-Alkyl, Aralkyl, Hetarylalkyl, Alkoxyalkyl, Aryloxyalkyl, Aryl, Hetaryl bedeutet, oder

$R^2$ + $R^4$  zusammen mit dem C, dessen Substituenten sie sind, einen $C_3$-$C_8$-Cycloalkylring bedeuten,

$R^5$  Wasserstoff, Alkyl, Aralkyl, Cycloalkyl, Hydroxy, Alkyloxy, $NH_2$, Alkylamino, Dialkylamino bedeutet,

$R^6$  Wasserstoff, Alkyl, Alkanoyl bedeutet,

$R^7$  Wasserstoff, Alkyl, Alkanoyl bedeutet,

$R^8$  Wasserstoff, Alkyl bedeutet,

Y  Alkyl, Alkenyl, Alkinyl, O, $S(O)_m$ (m = 0, 1, 2), alkylsubstituiertes N, Oxycarbonyl, Carbonyloxy, Oxycarbonylalkyl, Carbonyloxyalkyl, Oxyalkyloxy, Oxyalkenyl, Alkyloxy, Oxyalkyl, Thioalkyl, Azo, Carbonylamino, Aminocarbonyl, Aminocarbonyloxy, Oxyimino, Iminooxyalkyl bedeutet,

Z  Alkyl, Alkenyl, Alkinyl, Aryl, Aryl-Alkyl, Aryl-Alkenyl, Aryloxy-Alkyl, Alkoxyalkyl, Halogenalkyl, Alkoxycarbonyl, Hetaryl, Hetaryloxy, Aryloxy, Aryloxyalkoxy, Arylalkoxy, Alkoxy, Alkylthio, Arylthio bedeutet, wobei die genannten Reste gegebenenfalls substituiert sind,

X  Wasserstoff, Halogen, Nitro, Alkyl, Alkoxy oder Halogenalkyl bedeutet und

m  eine ganze Zahl zwischen 1 und 4 bedeutet,

und diese Verbindungen enthaltende Fungizide.

Die vorliegende Erfindung betrifft neue Oximetherderivate, ihre Herstellung und ihre Verwendung als Fungizide.

Es ist bekannt, Oximether z.B. den 1-Methoxyimino-1-(2-Phenoxymethylphenyl)-essigsäuremethylester als Fungizide zu verwenden (EP 253 213). Ihre Wirkung ist jedoch unbefriedigend.

Es wurde nun gefunden, daß neue Oximether der Formel I

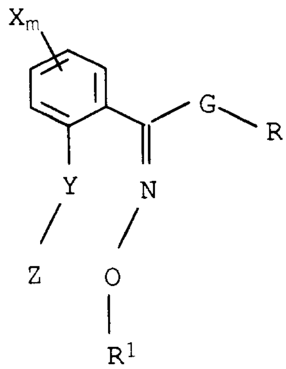

I,

in der

G 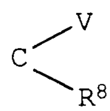 bedeutet,

V O-$R^6$, 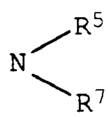 bedeutet,

R gegebenenfalls substituiertes $C_3$-$C_8$-Cycloalkyl, gegebenenfalls substituiertes $C_2$-$C_8$-Alkenyl, gegebenenfalls substituiertes $C_2$-$C_8$-Alkinyl, gegebenenfalls substituiertes Phenyl, gegebenenfalls substituiertes Hetaryl oder 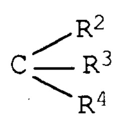 bedeutet,

$R^1$ Wasserstoff, $C_1$-$C_8$-Alkyl bedeutet,

$R^2$ Wasserstoff, $C_1$-$C_8$-Alkyl, Aralkyl, Hetarylalkyl, Alkoxyalkyl, Aryloxyalkyl, Aryl, Hetaryl bedeutet,

$R^3$ Wasserstoff, Methyl, -COO-A bedeutet,

A Wasserstoff, $C_1$-$C_8$-Alkyl bedeutet, oder für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$ Wasserstoff, $C_1$-$C_8$-Alkyl, Aralkyl, Hetarylalkyl, Alkoxyalkyl, Aryloxyalkyl, Aryl, Hetaryl bedeutet, oder

$R^2$ + $R^4$ zusammen mit dem C, dessen Substituenten sie sind, einen $C_3$-$C_8$-Cycloalkylring bedeuten,

$R^5$ Wasserstoff, $C_1$-$C_8$-Alkyl, $C_1$-$C_4$-Aralkyl, $C_3$-$C_6$-Cycloalkyl, Hydroxy, $C_1$-$C_4$-Alkyloxy, $NH_2$, $C_1$-$C_4$-Alkylamino, $C_1$-$C_4$-Dialkylamino bedeutet,

$R^6$ Wasserstoff, $C_1$-$C_8$-Alkyl, $C_1$-$C_4$-Alkanoyl bedeutet,

$R^7$ Wasserstoff, $C_1$-$C_8$-Alkyl, $C_1$-$C_4$-Alkanoyl bedeutet,

$R^8$ Wasserstoff, $C_1$-$C_8$-Alkyl bedeutet,

Y gegebenenfalls substituiertes $C_1$-$C_8$-Alkyl, gegebenenfalls substituiertes $C_2$-$C_8$-Alkenyl, $C_2$-$C_8$-Alkinyl, O, $S(O)_m$ (m = 0, 1, 2), N, das substituiert ist durch gegebenenfalls substituiertes $C_1$-$C_8$-Alkyl, Oxycarbonyl, Carbonyloxy, Oxycarbonyl-$C_1$-$C_8$-alkyl, Carbonyloxy-$C_1$-$C_8$-alkyl, $C_2$-$C_8$-Oxyalkyloxy, $C_2$-$C_8$-Oxyalkenyl, $C_1$-$C_8$-Alkyloxy, Oxy-$C_1$-$C_4$-alkyl, $C_1$-$C_8$-Thioalkyl, Azo, gegebenenfalls $C_1$-$C_8$-Alkylsubstituiertes Carbonylamino, gegebenenfalls $C_1$-$C_8$-alkylsubstituiertes Aminocarbonyl, gegebenenfalls $c_1$-$C_8$-alkylsubstituiertes Aminocarbonyloxy, Oxyimino, gegebenenfalls substituiertes Iminooxyalkyl bedeutet,

Z $C_1$-$C_{18}$-Alkyl, $C_2$-$C_{18}$-Alkenyl, $C_3$-$C_8$-Alkinyl, Aryl, Aryl-$C_1$-$C_{10}$-Alkyl, Aryl-$C_2$-$C_{10}$-Alkenyl, Aryloxy-$C_1$-$C_{10}$-Alkyl, $C_1$-$C_4$-Alkoxy-$C_1$-$C_{10}$-alkyl, $C_1$-$C_{10}$-Halogenalkyl, $C_1$-$C_4$-Alkoxycarbonyl, Hetaryl bedeutet,

Z bedeutet für den Fall, daß Y nicht Oxyalkyl, Oxyalkyloxy, Thioalkyl, -O-N=C-, -N=N, Oxycarbonyl, Oxycarbonyloxy, O oder S ist, zusätzlich noch Aryloxy, Aryloxy-$C_1$-$C_{10}$-alkoxy, Aryl-$C_1$-$C_{10}$-alkoxy, $C_1$-$C_{10}$-Alkoxy oder Hetaryloxy,

Z bedeutet für den Fall, daß Y nicht Oxyalkyl, Oxyalkyloxy, -O-N=C, -N=N-, Oxycarbonyl, Oxycarbonylalkyl oder O ist, zusätzlich noch $C_1$-$C_{18}$-Alkylthio oder Arylthio,

wobei die für Z genannten Reste gegebenenfalls substituiert sind durch Halogen, Cyano, Nitro, $C_1$-$C_4$-Alkyl, $C_2$-$C_4$-Alkenyl, $C_1$-$C_4$-Alkoxy, $C_1$-$C_4$-Halogenalkyl, $C_1$-$C_4$-Alkoxy-$C_1$-$C_4$-alkyl, $C_1$-$C_4$--dialkoxy-$C_1$-$C_4$-Alkyl, gegebenenfalls substituiertes O-$C_1$-$C_{10}$-Alkyloxyimino, gegebenenfalls substituiertes $C_2$-$C_{10}$-Alkanoyl, gegebenenfalls substituiertes Formyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten einen gegebenenfalls substituierten aromatischen oder heteroaromatischen Ring bilden können,

X gleich oder verschieden ist und Wasserstoff, Halogen, Nitro, gegebenenfalls substituiertes $C_1$-$C_4$-Alkyl, gegebenenfalls substituiertes $C_1$-$C_4$-Alkoxy oder gegebenenfalls substituiertes $C_1$-$C_4$-Halogenalkyl bedeutet und

m eine ganze Zahl zwischen 1 und 4 bedeutet

eine sehr gute fungizide Wirkung haben, die besser als die der bekannten Fungizide ist. Die neuen Verbindungen haben auch eine insektizide Wirkung. Die neuen Verbindungen der Formel I können bei ihrer Herstellung aufgrund der C==N-Doppelbindungen als E-Z-Isomerengemische anfallen, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.

R bedeutet bevorzugt Phenyl, Furyl, Thienyl, Cyclopropyl, Cyclobutyl, Cyclopentyl, Cyclohexyl, Vinyl oder

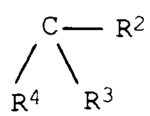

$R^1$ bedeutet bevorzugt Methyl, Ethyl, iso-Propyl, Propyl.

$R^2$ bedeutet bevorzugt Wasserstoff, Methyl, Ethyl, Propyl.

$R^3$ bedeutet bevorzugt Wasserstoff oder -CO-$OCH_3$.

$R^4$ bedeutet bevorzugt Wasserstoff, Methyl, Ethyl, Propyl.

$R^5$ bedeutet bevorzugt methyl, Ethyl, n-Propyl, iso-Propyl, Cyclopropyl.

$R^6$ bedeutet bevorzugt Wasserstoff, Methyl, Ethyl und Acetyl,

$R^7$ bedeutet bevorzugt Wasserstoff,

$R^8$ bedeutet bevorzugt Wasserstoff und Methyl,

Y bedeutet bevorzugt Methylen, Ethylen, Ethenylen, Ethenylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-$CH_2$, -O==C-, -C==N-O-, -N==O-, -N==-Gruppe oder O oder S.

Z bedeutet bevorzugt Wasserstoff, Halogen (z.B. Fluor, Chlor, Brom), $C_1$-$C_8$-Alkyl (z.B. Methyl, Ethyl, n- und iso-Propyl, n-, sec-, iso- und tert.-Butyl, 3-Methylbutyl, 2,2-Dimethylpropyl, 1,1-Dimethylpropyl, 3-Hexyl, n-Pentyl, n-Hexyl, n-Heptyl, n-Octyl, n-Nonyl, n-Decyl, n-Pentadecyl, n-Heptadecyl, 2,6-Dimethyl-hept-1-yl)-;Cyanmethyl;
$C_2$-$C_9$-Alkenyl (z.B. Vinyl, 2-Propen-2-yl, 1-Propen-1-yl, 2-Buten-2-yl, 2-Methyl-prop-1-en-1-yl, Allyl, 2-Buten-1-yl, 3-Methyl-2-buten-1-yl, 1,3-Pentadien-1-yl, 2,6-Dimethyl-1,5-heptadien-1-yl, 2,6-Dimethyl-hept-5-en-1-yl); $C_2$-Alkinyl (z.B. Ethinyl, 2-Phenylethinyl); $C_1$-$C_2$-Alkoxy-$C_1$-$C_2$-alkyl (Methoxymethyl, Ethoxymethyl, 1-Methoxyethyl), $C_3$-$C_6$-Cycloalkyl (z.B. Cyclopropyl, Cyclobutyl, Cyclopentyl, Cyclohexyl, 1-Methyl-cyclopropyl), wobei z.B. folgende substituierte Cyclopropylreste gemeint sind:
2,2-Dichlor-1-methyl-cyclopropyl (A1)
2,2-Dichlor-3,3-dimethyl-cyclopropyl (A2)
2,2,3,3-Tetramethylcyclopropyl (A3)
2-(2'-Methyl-1'-propenyl)-3,3-dimethylcyclopropyl (A4)
1-(2',2'-Difluorvinyl)-3,3-dimethylcyclopropyl (A5)
2-(2'-2,'-Dichlorvinyl)-3-3-dimethylcyclopropyl (A6)
2-(2',2'-Dibromvinyl)-3,3-dimethylcyclopropyl (A7)
2-Phenylcyclopropyl (A8)
2-(4'-Chlorphenyl)-cyclopropyl A9)
2,2-Dichlor-3-phenylcyclopropyl (A10)
2-Carbomethoxy-cyclopropyl (A11)
und Halogen-$C_1$-$C_2$-alkyl (z.B. Chlormethyl, 1-Chlorethyl, Dichlormethyl, Trichlormethyl, Brommethyl, Trifluormethyl), Phenyl, substituiertes Phenyl, wie Halogenphenyl (z.B. 2-Fluor-, 3-Fluor-, 4-Fluor-, 6-Fluor-, 2-Chlor-, 2,4-Difluor-, 2,6-Difluor-, 2,3,4,5,6-Pentafluor-, 2-Chlor-, 3-Chlor-, 4-Chlor-, 2,5-Dichlor-, 2,5-Dichlor-, 2,6-Dichlor-, 3,4-Dichlor-, 3,5-Dichlor-, 2,4,5-Trichlor-, 2,3,4,5,6Pentachlor-, 2-Brom-, 3-Brom-, 4-Bromphenyl); $C_1$-$C_4$-Alkylphenyl (z.B. 2-Methyl-, 3-Methyl-, 2,3-Dimethyl-, 2,4-Dimethyl-, 2,5-Dimethyl-, 2,6-Dimethyl-, 3,4-Dimethyl-, 3,5-Dimethyl-, 2,4,6-Trimethyl-, 4-t-Butyl-Phenyl); Arylphenyl (z.B. 2-Phenyl-, 4-Phenylphenyl); Halogen-$C_1$-alkylphenyl (z.B. 2-Trifluor-, 3-Trifluor-, 4-Trifluormethylphenyl); $C_1$-$C_4$-Alkoxyphenyl (z.B. 2-Methoxy-, 3-Methoxy-, 4-Methoxy-, 3,4-Dimethoxy-, 3,4,5-Trimethoxy-, 4-t-Butoxyphenyl); 2-, 3-, 4-Phenoxyphenyl); $C_1$-$C_4$-Alkanoylphenyl (z.B. 4-Acetyl-, 3-Acetyl-, 2-Acetyl-, 4-Acetyl-2-methylphenyl); Formylphenyl (z.B. 2-Formyl, 3-Formyl-, 4-Formylphenyl); $C_1$-$C_4$-Dialkoxy-$C_1$-$C_4$-alkylphenyl [z.b. 4-(1,1-Dimethoxyethyl), 4-(Dimethoxymethyl), 3-(1,1-Dimethoxyethyl), 3-(Dimethoxymethyl), 4-(1,1-Dimethoxyethyl)-2-methylphenyl], $C_1$-$C_{10}$-O-Alkyloxyiminophenyl (z.B. 4-[1-(O-Ethyloxyimino)ethyl], 4-(O-Ethyloxyiminomethyl), 3-[1-(O-Ethyloxyimino)ethyl], 3-(O-Ethyloxyiminomethyl), 4-[1-(O-Ethyloxyimino)ethyl]-2-methyl, 4-[1-(O-Hexyloxyimino)ethyl], 4-(O-Hexyloxyiminomethyl), 3-[1-(O-Hexyloxyimono)ethyl], 3-(O-Hexyloxyiminomethyl), 4-[1-(O-Hexyloxyimino)ethyl]-methyl, 4-[1-(O-iso-Butyloxyimino)ethyl], 4-(O-iso-Butyloxyimino)ethyl]-2-methylphenyl);
$C_2$-$C_{10}$-Alkenyloxyiminophenyl (z.B. 4-(1-(O-2-Buten-1-oxyimino)ethyl], 4-(O-2-Buten-1-oxyiminomethyl), 3-[1-(O-2-Buten-1-oxyimino)ethyl], 3-(O-2-Buten-1-oxyiminomethyl), 4-(1-(O-2-Buten-1-oxyimino)ethyl]-2-methylphenyl); $C_1$-$C_{10}$-Arylalkyloxyiminophenyl (z.B. 4-[1-(O-Benzyloxyimino)-ethyl], 4-(O-Benzyloxylminomethyl), 3-[1-(O-Benzyloxyimino)ethyl], 3-(O-Benzyloxyiminomethyl), 4-(1-(O-Benzyloxyimino)ethyl)-2-methylphenyl, substituiertes Benzyl (z.B. Halogenbenzyl, 2-Fluor-, 3-Fluor-, 4-Fluor-, 2-Chlor-, 3-Chlor-, 4-Chlor-, 2-Chlor-, 6-Fluor-, 2,4-Dichlor-, 2,6-Dichlor-, 3,5-Dichlor-, 2,4,6-Trichlor-, 2-Brom-, 3-Brom-, 4-Brom-, 2-Methyl-, 3-Methyl-, 4-Methyl-, 4-t-Butyl-, 2,4-Dimethyl-, 2,6-Dimethyl-, 2,4,6-Trimethyl-, 2-Methoxy-, 3-Methoxy-, 4-Methoxy-, 2-Trifluormethyl-, 3-Trifluormethyl-, 4-Trifluormethyl-, 4-t-Butoxy-, 4-Phenoxy-, 4-Phenylbenzyl-, -Methyl--ethyl-, -iso-Propyl-, -Hydroxy-, 2-Methoxy-hydroxy-, 4-Methoxy-hydroxy-, 3,4-Dimethoxy--hydroxy-, 2-Methoxy-methoxy-, 4-Methoxy-methoxy-, 3,4-Dimethoxy-methoxybenzyl); gegebenenfalls substituiertes Phenethyl (z.B. Phenyl-, 1-Methyl-2-phenyl-, 2-(para-t-Butylphenyl-), 2-(para-t-Butylphenyl)-1-Methyl-, 2-(ortho-Chlorphenyl)-, 2-(meta-Chlorphenyl)-, 2-(para-Chlorphenyl)-ethyl); gegebenenfalls substituiertes Styryl (z.B. Styryl; 2'-Chlor-, 3'-Chlor-, 4'-Chlor-, 2',4'-Dichlor-, 2'-Fluor-, 4'-Fluor-, 2'-Methyl-, 4'-Methyl-, 4'-t-Butyl-, 2'-Methoxy-, 4'-Methoxy-, 4'-Phenoxy-styryl); Phenyl-$C_3$-C6-alkyl (z.B. 3-Phenylpropyl, 2-Methyl-3-Phenylpropyl, 4-Phenylbutyl, 5-Phenylpentyl, 6-Phenylhexyl, 3-(4'-t-Butylphenyl)-2-Methylpropyl); Aryloxy, (wenn v = o, s, Oxycarbonyl, Oxyalkoxy, Oxyimino, Thioalkyl, -N = = N-, Oxycarbonylalkyl, Oxyalkyl), wie substituiertes Phenoxy (z.B. 2-Chlor-, 3-Chlor-, 4-Chlor-, 2-Methyl-, 4-Methyl-, 2-Methoxy-, 4-Methoxy-, 2-Trifluormethyl-, 4-Trifluormethylphenoxy); Aryloxy-$C_1$-C6-alkyl, wie gegebenenfalls substituiertes Phenoxymethyl (z.B. 2-Chlor-, 2-Methyl-, 4-Methyl-, 2-Methoxy-, 4-Methoxy-, 4-t-Butyl-phenoxymethyl); gegebenenfalls substituiertes Phenoxyethyl (z.B. 2-Chlor-, 4-Chlor-, 4-Fluor-, 2-Methyl-, 4-Methyl-, 2-Methoxy-, 4-Methoxy-, 4-t-Butyl-phenoxyethyl), gegebenenfalls substituiertes $C_3$-$C_6$-Phenoxyalkyl (z.B. 3-Phenoxypropyl, 3-(ortho-Chlorphenoxy)propyl, 3-(para-Chlor-phenoxy)propyl, 4-Phenoxy- butyl, 4-(ortho-Chlorphenoxy)-butyl, 4-(para-Chlorphenoxybutyl), 5-Phenoxypentyl, 5-(ortho-Chlorphenoxy)pentyl, 5-(para-Chlorphenoxy)pentyl, 6-Phenoxyhexyl); Phenoxyethoxy (wenn v = O, S, Oxycarbonyl, Oxyalkoxy, Oxyimino, Thioalkyl, -N = = N-, oxycarbonylalkyl, Oxyalkyl), Methoxycarbonyl, t-Butoxycarbonyl; gegebenenfalls substituiertes Heteroaryl (z.B. Furyl, 2-Furyl, 3-Furyl. 5-Nitro-2- und 3-Furyl, 5-Chlor-2- und 3-Furyl, Benzfuran-2- und 3-yl, Thienyl, 2-Thienyl, 3-Thienyl, 5-Nitro-2- und 3-thienyl, 5-Chlor-2- und 3-thienyl, Benzothienyl-2- und 3-yl.

N-Methyl-pyrrol-2- und 3-yl, N-Methyl-pyrazol-3-, 4- und 5-yl, N-Methylimidazol-2-, 4- und 5-yl, 1-Methyl-1,2,3-triazol-4- und 5-yl, 1-Methyl-1,2,4-triazol-3- und 5-yl, 1-Methyl-tetrazol-5-yl,Isoxazol-3-, 4- und 5-yl, Benzisoxazol-3-yl, Benzoxazol-2-yl, Oxazol-2-, 4- und 5-yl, Thiazol-2-, 4- und 5-yl, Benzothiazol-2-yl, Benzisothiazol-3-yl, Isothiazol-3-, 4- und 5-yl, 1,2,3-Thiadiazol-4-yl, 1,2,4-Thiadiazol-5-yl, 1,3,4-Thiadiazol-3-yl, 1,3-Thiazolo-[4,5-b]-pyridin-2-yl; substituiertes Hetarylalkenyl (z.B. 2-(2'- und 3'-Furyl)-, 2-(5'-Nitro-2'- und 3'-furyl)-, 2-(2'- und 3'-Thienyl)-, 2-(5'-Nitro-2'- und 3'-thienyl)-ethenyl).

Weitere bevorzugte Verbindungen sind Oximether der Formel I in der

G

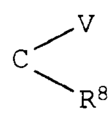

bedeutet,

V O-$R^6$ bedeutet,

R $C_3$-$C_8$-Cycloalkyl, gegebenenfalls substituiertes $C_2$-$C_8$-Alkenyl, gegebenenfalls substituiertes Phenyl, gegebenenfalls substituiertes Hetaryl oder

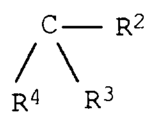

bedeutet,

$R^1$ Wasserstoff, $C_1$-$C_8$-Alkyl bedeutet,

$R^2$ Wasserstoff, $C_1$-$C_8$-Alkyl, $C_3{}^{-C}{}_8$-Cycloalkyl oder gegebenenfalls durch $C_1$-$C_4$-Alkyl, $C_1$-$C_4$-Alkoxy oder Halogen substituiertes Phenyl bedeutet,

$R^3$ Wasserstoff, Methyl, Cyano, -COO-A- bedeutet,

A Wasserstoff, Methyl, Ethyl, iso-Propyl, Propyl, n-Butyl, sec-Butyl, tert-Butyl bedeutet oder für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$ Wasserstoff, $C_1$-$C_8$-Alkyl, $C_3$-$C_8$-Cycloalkyl oder gegebenenfalls durch $C_1$-$C_4$-Alkyl, $C_1$-$C_4$-Alkoxy oder Halogen substituiertes Phenyl bedeutet oder $R^2$ + $R^4$ zusammen mit dem C, dessen Substituenten sie sind, einen $C_3$-$C_8$-Cycloalkylring bedeuten,

$R^6$ Wasserstoff, $C_1$-$C_8$-Alkyl, $C_1$-$C_4$-Alkanoyl bedeutet,

$R^8$ Wasserstoff, $C_1$-$C_8$-Alkyl bedeutet,

Y Methylen, Ethylen, Ethenylen, Ethenylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C = = N-O-cH$_2$, -O- = = C-, -C = = N-O-, -N = = N-Gruppe oder O oder S bedeutet,

Z ein geradkettiges oder verzweigtes $C_1$-$C_{10}$-Alkyl, $C_2$-$C_{10}$-Alkenyl, $C_1$-$C_{10}$-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrazolyl, Benzoimidazolyl, Indolyl, Isoindolyl, Benzothiophenyl, Isobenzothiophenyl, Benzofuran, Isobenzofuranyl, oder nur, wenn Y nicht Oxymethylen, Oxyethylen, Oxycarbonyl, Oxacyrbonylmethylen, Thiomethylen, Thioethylen, -O-N = = C-, -N = = N-, O, S ist, ein geradkettiges oder verzweigtes $C_1$-$C_{10}$--alkyloxy, $C_1$-$C_{10}$-Alkenyloxy, Phenyloxy, Pyridinyloxy, Pyridazinyloxy, Pyrimidinyloxy, Pyrazinyloxy, Chinolyloxy, Isochinolyloxy, Naphthyridyloxy, Chinoxalyloxy, Thienyloxy, Furyloxy, Pyrrolyloxy, Oxazolyloxy, Isoxazolyloxy, Pyrazolyloxy, Imidazolyloxy, Thiazolyloxy, Isothiazolyloxy, Benzothiazolyloxy, Benzoisothiazolyloxy, Benzoxyzolyloxy, Benzoxazolyloxy, Benzoisoxazolyloxy, Benzopyrrazolyloxy, Benzoimidazolyloxy, Indolyloxy, Isoindolyloxy, Benzothiophenyloxy, Isobenzothiophenloxy, Benzofuranyloxy, Isobenzofuranyloxy, oder nur, wenn Y nicht Oxymethylen, Oxyethylen, Oxycarbonyl, Oxycarbonylmethylen,

-O-N = = C-, -N = = N-, O ist, ein geradkettiges oder verzweigtes $C_1$-$C_{10}$-Alkylthio, $C_2$-$C_{10}$-Alkenylthio, Phenylthio, Pyridinylthio, Pyridazinylthio, Pyrimidinylthio, Pyrazinylthio, Chinolylthio, Isochinolylthio, Naphthyridylthio, Chinoxalylthio, Thienylthio, Furylthio, Pyrrolylthio, Oxazolylthio, Isoxazolylthio, Pyrazolylthio, Imidazolylthio, Thiazolylthio, Isothiazolylthio, Benzothiazolylthio, Benzoisothiazolylthio, Benzoxazolylthio, Benzoisoxazolylthio, Benzopyrazolylthio, Benzoimidazolylthio, Indolylthio, Isoindolylthio, Benzothiophenylthio, Isobenzothiophenylthio, Benzofuranylthio, Isobenzofuranylthio bedeutet, wobei die für Z genannten Reste gegebenenfalls substituiert sind mit Halogen, Cyano, geradkettigem oder verzweigtem $C_1$-$C_4$-Alkyl, $C_2$-$C_4$-Alkenyl, $C_2$-$C_4$-Alkinyl, Hydroxyimino, $C_1$-$C_4$-Carboxylalkoxy, $C_1$-$C_4$-Alkanoyl, $C_1$-$C_4$-Alkoxyalkyl, $C_1$-$C_4$-Dialkoxyalkyl, O-Alkyloxyimino, O-Alkenyloxyimino, O-Arylalkyloxyimino, $C_1$-$C_4$-Dialkoxy, $C_1$-$C_4$-Alkyl, Formyl,

X Wasserstoff, Halogen, Cyano, geradkettiges oder verzweigtes $C_1$-$C_4$-Alkyl, $C_1$-$C_4$-Halogenalkyl, $C_1$-$C_4$-Alkoxy bedeutet und

m eine ganze Zahl zwischen 1 und 4 bedeutet.

Weitere bevorzugte Verbindungen sind Oximether der Formel I in der

G

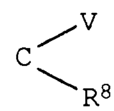

bedeutet,

V

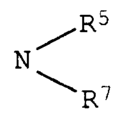

bedeutet,

R $C_3$-$C_8$-Cycloalkyl, gegebenenfalls substituiertes $C_2$-$C_8$-Alkenyl, gegebenenfalls substituiertes $C_2$-$C_8$-Alkinyl, gegebenenfalls substituiertes Phenyl, gegebenenfalls substituiertes Hetaryl oder

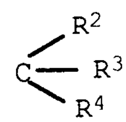

bedeutet,

$R^1$ Wasserstoff, $C_1$-$C_6$-Alkyl bedeutet,

$R^2$ Wasserstoff, $C_1$-$C_8$-Alkyl, $C_3$-$C_8$-Cycloalkyl oder gegebenenfalls durch $C_1$-$C_4$-Alkyl, $C_1$-$C_4$-Alkoxy oder Halogen substituiertes Phenyl bedeutet,

$R^3$ Wasserstoff, Methyl, Cyano, -COO-A- bedeutet,

A Wasserstoff, Methyl, Ethyl, iso-Propyl, Propyl, n-Butyl, sec-Butyl, tert-Butyl bedeutet oder für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$ Wasserstoff, $C_1$-$C_8$-Alkyl, $C_3$-$C_8$-Cycloalkyl oder gegebenenfalls durch $C_1$-$C_4$-Alkyl, $C_1$-$C_4$-Alkoxy oder Halogen substituiertes Phenyl bedeutet oder

$R^2$ + $R^4$ zusammen mit dem C, dessen Substituenten sie sind, einen $C_3$-$C_8$-Cycloalkylring bedeuten,

$R^5$ Wasserstoff, $C_1$-$C_8$-Alkyl, $C_1$-$C_4$-Aralkyl, $C_3$-$C_6$-Cycloalkyl, Hydroxy, $C_1$-$C_4$-Alkyloxy, $NH_2$, $C_1$-$C_4$-Alkylamino, $C_1$-$C_4$-Dialkylamino bedeutet,

$R^7$ Wasserstoff, $C_1$-$C_8$-Alkyl, $C_1$-$C_4$-Alkanoyl bedeutet,

$R^8$ Wasserstoff, $C_1$-$C_8$-Alkyl bedeutet,

Y Methylen, Ethylen, Ethenylen, Ethenylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C = = N-O-c$H_2$, -O- = = C-, -C = = N-O-, -N = = N-Gruppe oder O oder S bedeutet,

Z ein geradkettiges oder verzweigtes $C_1$-$C_{10}$-Alkyl, $C_2$-$C_{10}$-Alkenyl, $C_1$-$C_{10}$-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isoxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrazolyl, Benzoimidazolyl, Indolyl, Isoindolyl, Benzothiophenyl, Isobenzothiophenyl, Benzofuran, Isobenzofuranyl, oder nur, wenn Y nicht Oxymethylen, Oxyethylen, Oxycarbonyl, Oxacyrbonylmethylen, Thiomethylen, Thioethylen, -O-N = = C-, -N = = N-, O, S ist, ein geradkettiges oder verzweigtes $C_1$-$C_{10}$--alkyloxy, $C_1$-$C_{10}$-Alkenyloxy, Phenyloxy, Pyridinyloxy, Pyridazinyloxy, Pyrimidinyloxy, Pyrazinyloxy, Chinolyloxy, Isochinolyloxy, Naphthyridyloxy, Chinoxalyloxy, Thienyloxy, Furyloxy, Pyrrolyloxy, Oxazolyloxy, Isoxazolyloxy, Pyrazolyloxy, Imidazolyloxy, Thiazolyloxy, Isothiazolyloxy, Benzothiazolyloxy, Benzoisothiazolyloxy, Benzoxyzolyloxy, Benzoxazolyloxy, Benzoisoxazolyloxy, Benzopyrrazolyloxy, Benzoimidazolyloxy, Indolyloxy, Isoindolyloxy, Benzothiophenyloxy, Isobenzothiophenloxy, Benzofuranyloxy, Isobenzofuranyloxy, oder nur, wenn Y nicht Oxymethylen, Oxyethylen, Oxycarbonyl, Oxycarbonylmethylen, -O-N = = C-, -N = = N-, O ist, ein geradkettiges oder verzweigtes $C_1$-$C_{10}$-Alkylthio, $C_2$-$C_{10}$-Alkenylthio, Phenylthio, Pyridinylthio, Pyridazinylthio, Pyrimidinylthio, Pyrazinylthio, Chinolylthio, Isochinolylthio, Naphthyridylthio, Chinoxalylthio, Thienylthio, Furylthio, Pyrrolylthio, Oxazolylthio, Isoxazolylthio, Pyrazolylthio, Imidazolylthio, Thiazolylthio, Isothiazolylthio, Benzothiazolylthio, Benzoisothiazolylthio, Benzoxazolylthio, Benzoisoxazolylthio, Benzopyrazolylthio, Benzoimidazolylthio, Indolylthio, Isoindolylthio, Benzothiophenylthio, Isobenzothiophenylthio, Benzofuranylthio, Isobenzofuranylthio bedeutet, wobei die für Z genannten Reste gegebenenfalls substituiert sind mit Halogen, Cyano, geradkettigem oder verzweigtem $C_1$-$C_4$-Alkyl, $C_2$-$C_4$-Alkenyl, $C_2$-$C_4$-Alkinyl, Hydroxyimino, $C_1$-$C_4$-Carboxylalkoxy, $C_1$-$C_4$-Alkanoyl, $C_1$-$C_4$-Alkoxyalkyl, $C_1$-$C_4$-Dialkoxyalkyl, O-Alkyloxyimino, O-Alkenyloxyimino, O-Arylalkyloxyimino, $C_1$-$C_4$-Dialkoxy, $C_1$-$C_4$-Alkyl, Formyl,

X Wasserstoff, Halogen, Cyano, geradkettiges oder verzweigtes $C_1$-$C_4$-Alkyl, $C_1$-$C_4$-Halogenalkyl, $C_1$-$C_4$-Alkoxy bedeutet und

m eine ganze Zahl zwischen 1 und 4 bedeutet.

X Wasserstoff, Halogen, Cyano, geradkettiges oder verzweigtes $C_1$-$C_4$-Alkyl, $C_1$-$C_4$-Halogenalkyl, $C_1$-$C_4$-Alkoxy bedeutet und

m eine ganze Zahl zwischen 1 und 4 bedeutet.

Die Verbindungen der Formel Ib, Ic lassen sich herstellen, indem man eine Phenylglyoxylsäuremethylester-O-alkyloxim (EP 253 213) der Formel II

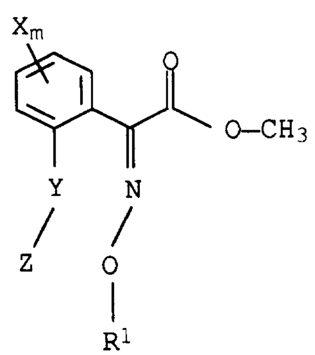

II,

in der $R^1$, Y, Z, X, m die oben genannte Bedeutung haben, mit einer Verbindung der Formel III

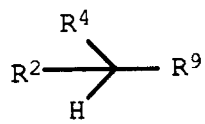

III,

in der $R^2$ und $R^4$ die oben genannte Bedeutung haben und $R^9$ gleich -CN oder -COO-A bedeutet, wobei A die oben genannte Bedeutung hat, unter basischen Bedingungen umsetzt (E.V.P. Tao, G.S. Staten Org.Prep.Proc.Int. Briefs 17 (1985) 235).

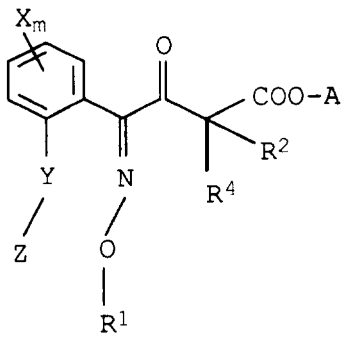

Ib

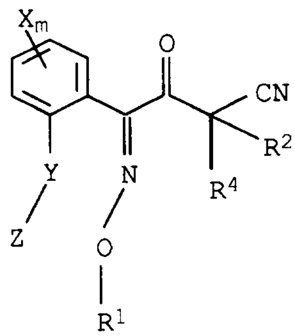

Ic

Die Verbindungen Ib lassen sich weiter zu Verbindungen des Typs Ia umsetzen (S. Takei, Y. Kawano, Tetrahedron Lett. 49 (1975) 4389).

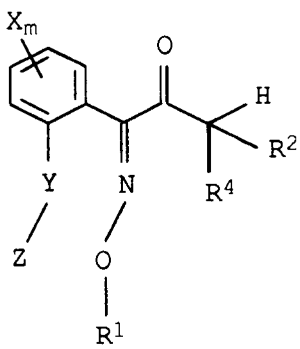

Ia

Ausgehend von Verbindungen der Formel II lassen sich die Verbindungen der Formel Id auf einem anderen Weg gewinnen. In bekannter Weise kann man die Verbindungen der Formel IV leicht herstellen (S. Nahm, S.M. Weinreb, Tetrahedron Lett. 39 (1981) 3815).

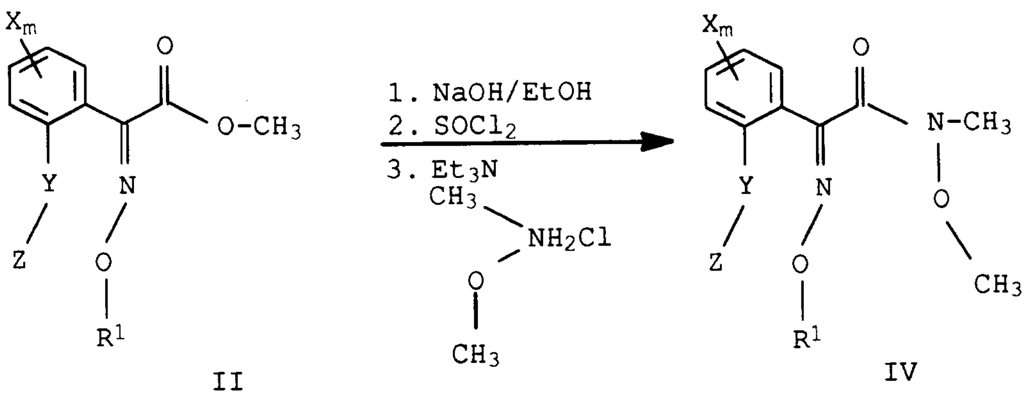


II

IV

Die weitere Umsetzung der Amide IV mit Organolithiumreagenzien (S. Nahm, S.M. Weinreb, Tetrahedron Lett. 39 (1981) 3815), liefert die gewünschten α-Oxyiminoketone der Formel Id.

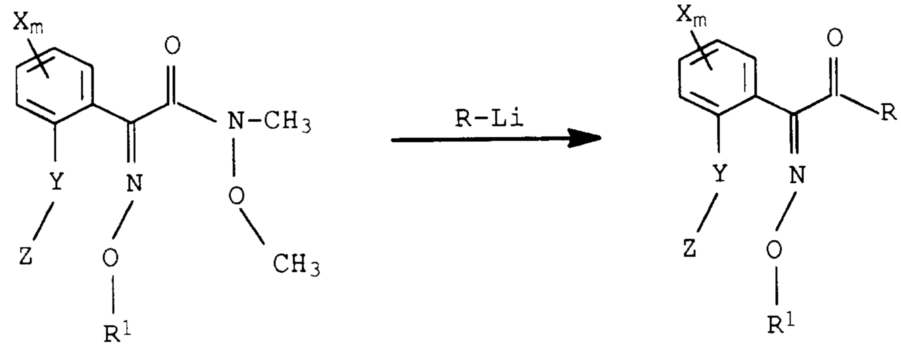


IV Id

Es ist auch bereits bekannt, daß Verbindungen des Typs Id mit Aminen, Hydroxylaminen und Hydrazinen reagieren und Verbindungen des Typs Ie liefern (S. 804-805 Advanced Organic Chemistry J. March dritte Auflage)

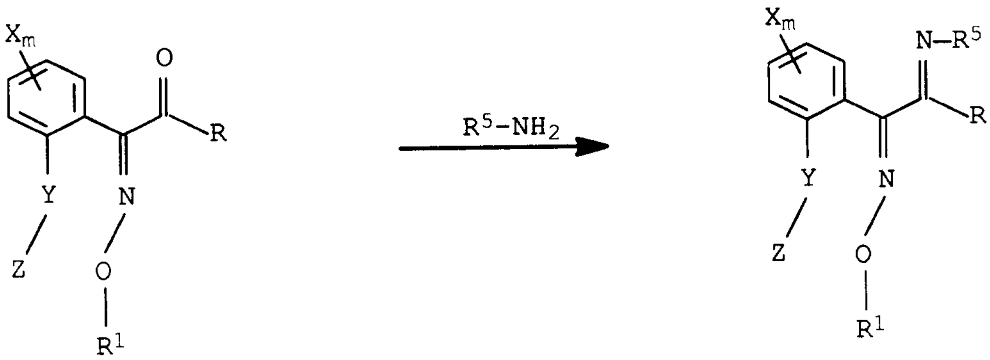


Id Ie

Weiterhin ist auch bekannt, daß Imine, Oxime und Hydrazone des Typs Ie, sowie Ketone des Typs Id, von verschiedenen Reduktionsmitteln reduziert werden können (S. 694/814-815 "Advanced Organic Chemistry", J. March, dritte Auslage). Die Reaktion liefert bei der Anwendung von Ketonen Id die entsprechenden Alkohole If und bei der Anwendung von Ketonen Id die entsprechenden Alkohole If und bei der Anwendung von Iminen, Oximen oder Hydrazonen des Typs Ie die entsprechenden Amine, Hydroxylamine und Hydrazine Ig entstehen.

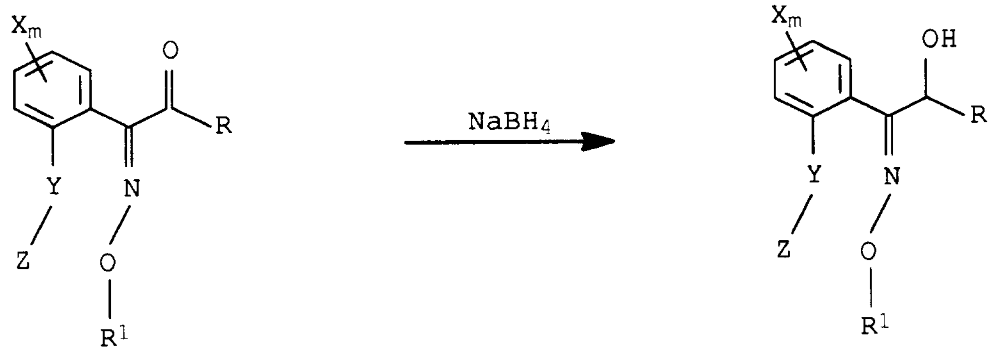


Id If

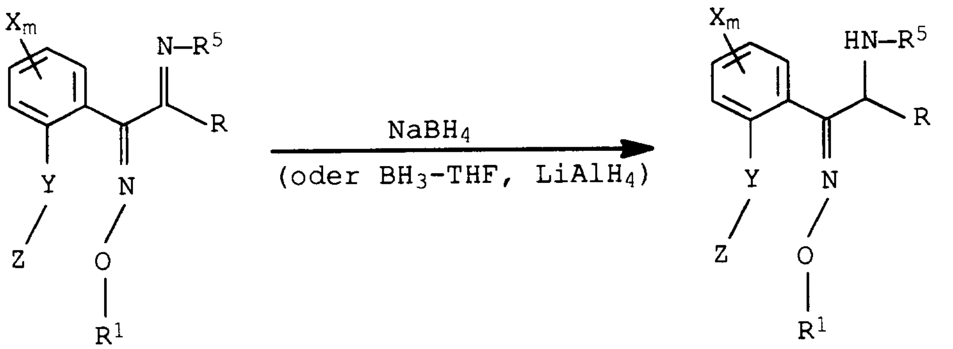


Ie Ig

Auf bekannte Weise lassen sich die Verbindungen If und Ig weiter alkylieren oder acylieren (S. 342/343/347 "Advanced Organic Chemistry", J. March, dritte Auflage, sowie S. 257 "Organikum", Fünfzehnte Auflage, sowie S. 295-324 "Houben-Weyl, Methoden der organischen Chemie" Bd. IV/3, H. Meerwein 1965) und liefern die Verbindungen Ih und Ii.

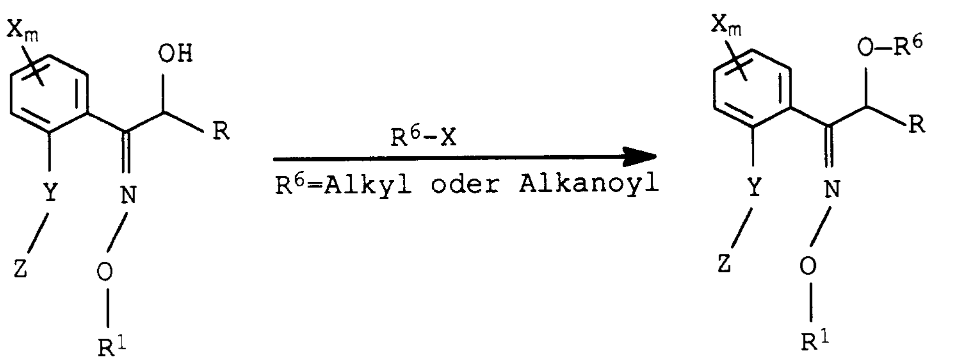


If Ih

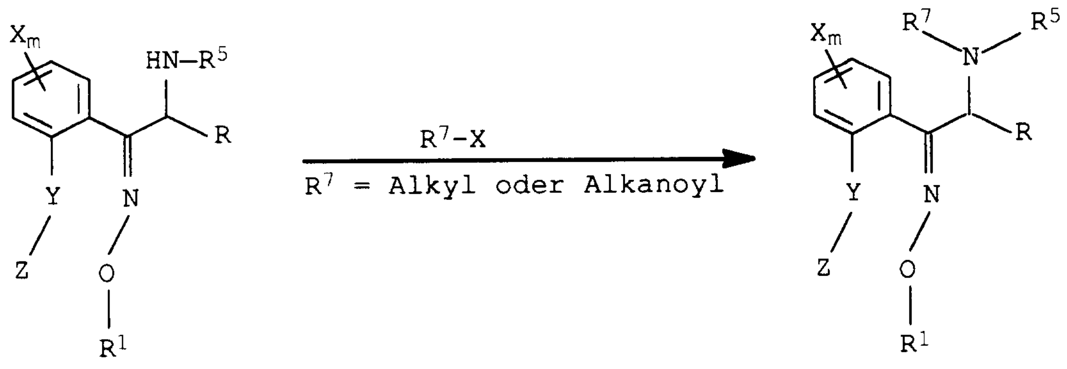


Ig Ii

Die Herstellung der neuen Verbindungen und der Zwischenprodukte wird durch die folgenden Beispiele und Vorschriften erläutert:

Herstellungsbeispiele

Beispiel 1

1-Methoxyimino-1-[2'-(2-methylphenoxymethyl)phenyl]-propan-2-ol (Verbindung Nr. 891)

5 g (17 mmol) 1-Methoxyimino-1-[2'-(2-methylphenoxymethyl)phenyl]-propan-2-on wird in Ethanol gelöst und 0,7 g $NaBH_4$ bei Raumtemperatur zugegeben. Das Gemisch wird über Nacht bei Raumtemperatur weitergerührt, das Lösungsmittel eingeengt, in tert-Butylmethylether aufgenommen, dreimal mit Wasser gewaschen, getrocknet und eingeengt. Das Rohprodukt wird durch Säulenchromatographie gereinigt (Cyclohexan/Essigsäureethylester 5:5) und liefert 3,8 g (76 %) Verbindung Nr. 891.
$^1$-H-NMR ($CDCl_3$): 7,70-6,75 (m,8H); 4,95 (m,2H); 4,65 (m,1H); 3,85 (s,3H); 2,95 (m,1H); 2,25 (s,3H); 1,35 (d,3H).

Beispiel 2

2-Methoxy-methoxyimino-1-[2'-(2-methylphenoxymethyl)phenyl]-propan (Verbindung Nr. 946)

0,27 g 50 %ige NaH (5,5 mmol) wird ein Pentan gewaschen und in 10 ml THF mit 0,7 g Dimethylsulfat vorgelegt. Eine Lösung von 1,5 g (5 mmol) Produkt (16) in 10 ml THF wird bei Raumtemperatur zugetropft und das Gemisch 3 Stunden weitergerührt. Wasser und Diethylether werden zugegeben, die organische Phase mit Natronlauge gewaschen, getrocknet und eingeengt. Man erhält 1,6 g (99 %) Verbindung Nr. 946.
$^1$-H-NMR ($CDCl_3$): 7,70-6,75 (m,8H); 4,95 (m,2H); 4,15 (m,1H); 3,90 (s,3H); 3,45 (m,1H); 2,30 (s,3H); 1,20 (d,3H).

In entsprechender Weise werden die in der folgenden Tabelle aufgeführten Verbindungen hergestellt.

Tabelle 1

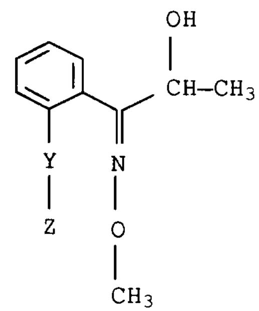

| Nr. | Y | Z | Fp. [°C] oder IR($cm^{-1}$) |
|---|---|---|---|
| 890 | $-CH_2O$ | -Ph | |
| 891 | - | -(2-Me)Ph | |
| 892 | - | -(2,4-DiMe)Ph | |
| 893 | - | -(4-Cl,2-Me)Ph | |
| 894 | - | -(4-Cl)Ph | |
| 895 | - | -(4-tBu,2 Me)Ph | |
| 886 | - | -(2,3,5-TriMe)Ph | |
| 897 | - | -(4-Cyclohexyl,2-Me)Ph | |
| 898 | - | -(4-Phenyl)Ph | |
| 899 | - | -(5-Me,2-iPr)Ph | |
| 900 | - | -(2,5-DiMe)Ph | |

| Nr. | Y | Z | Fp. [°C] oder IR($cm^{-1}$) |
|---|---|---|---|
| 901 | - | -(2,5-DiEt)Ph | |
| 902 | $-CH_2-O-\overset{O}{\overset{\|}{C}}$ | -(2-Me)Ph | |
| 903 | - | -[1-(4-ClPhenyl)]cyclopropyl | |
| 904 | - | -[1-(3,4-DiClPhenyl)]cyclopropyl | |
| 905 | - | -[1-(4-Methoxyhenyl)]cyclopropyl | |
| 906 | - | (1-Methyl)cyclopropyl | |
| 907 | - | -[2,2-DiMethyl,3-(2,2'-DiBrom-Ethylen)]cyclopropyl | |
| 908 | $-CH_2$ | -H | |
| 909 | - | -Br | |
| 910 | $-CH_2$ | -4-(1-oxo)ethyl-Ph | |
| 911 | - | -4[1-(2-propenoxyimino)]Ethyl-Ph | |
| 912 | - | -4(1-Ethyloxyimino)Ethyl-Ph | |
| 913 | - | -4(1-Benzyloxyimino)Ethyl-Ph | |
| 914 | - | -4(2-propenoxyimino)Methyl-Ph | |
| 915 | - | -4(Ethyloxyimino)Methyl-Ph | |
| 916 | - | -4(Benzyloxyimino)Methyl-Ph | |

Tabelle 1 (Fortsetzung)

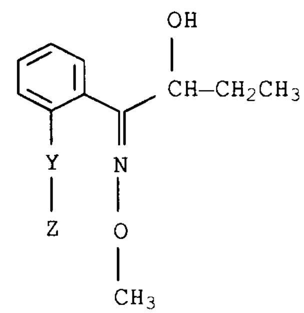

| Nr. | Y | Z | Fp. [°C] oder IR($cm^{-1}$) |
|---|---|---|---|
| 917 | $-CH_2O$ | -Ph | |
| 918 | – | -(2-Me)Ph | NMR: 7,70-6,70(m,8H); 4,95(m,2H); 4,45(m,1H); 3,90(s,3H); 2,30(s,3H); 1,60(m,2H); 1,00(t,3H) ppm |
| 920 | – | -(2,4-DiMe)Ph | |
| 921 | – | -(4-Cl,2-Me)Ph | |
| 922 | – | -(4-Cl)Ph | |
| 923 | – | -(4-tBu,2 Me)Ph | |
| 924 | – | -(2,3,5-TriMe)Ph | |
| 925 | – | -(4-Cyclohexyl,2-Me)Ph | |
| 926 | – | -(4-Phenyl)Ph | |

| Nr. | Y | Z | Fp. [°C] oder IR($cm^{-1}$) |
|---|---|---|---|
| 927 | - | -(5-Me,2-iPr)Ph | |
| 928 | - | -(2,5-DiMe)Ph | |
| 929 | - | -(2,5-DiEt)Ph | |
| 930 | $-CH_2-O-C(=O)$ | -(2-Me)Ph | |
| 931 | - | -[1-(4-ClPhenyl)]cyclopropyl | |
| 932 | - | -[1-(3,4-DiClPhenyl)]cyclopropyl | |
| 933 | - | -[1-(4-Methoxyhenyl)]cyclopropyl | |
| 934 | - | (1-Methyl)cyclopropyl | |
| 935 | - | -[2,2-DiMethyl,3-(2,2'-DiBrom-Ethylen)]cyclopropyl | |
| 936 | $-CH_2$ | -H | |
| 937 | - | -Br | |
| 938 | $-CH_2$ | -4-(1-oxo)ethyl-Ph | |
| 939 | - | -4[1-(2-propenoxyimino)]Ethyl-Ph | |
| 940 | - | -4(1-Ethyloxyimino)Ethyl-Ph | |
| 941 | - | -4(1-Benzyloxyimino)Ethyl-Ph | |
| 942 | - | -4(2-propenoxyimino)Methyl-Ph | |
| 943 | - | -4(Ethyloxyimino)Methyl-Ph | |
| 944 | - | -4(Benzyloxyimino)Methyl-Ph | |

Tabelle 1 (Fortsetzung)

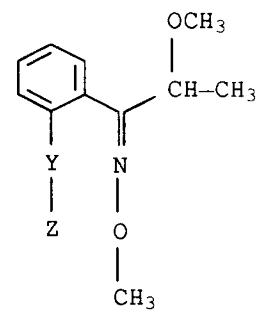

| Nr. | Y | Z | Fp. [°C] oder IR($cm^{-1}$) |
|---|---|---|---|
| 945 | $-CH_2O$ | -Ph | |
| 946 | - | -(2-Me)Ph | |
| 947 | - | -(2,4-DiMe)Ph | |
| 948 | - | -(4-Cl,2-Me)Ph | |
| 949 | - | -(4-Cl)Ph | |
| 950 | - | -(4-tBu,2 Me)Ph | |
| 951 | - | -(2,3,5-TriMe)Ph | |
| 952 | - | -(4-Cyclohexyl,2-Me)Ph | |
| 953 | - | -(4-Phenyl)Ph | |
| 954 | - | -(5-Me,2-iPr)Ph | |
| 955 | - | -(2,5-DiMe)Ph | |

| Nr. | Y | Z | Fp. [°C] oder IR($cm^{-1}$) |
|---|---|---|---|
| 956 | – | -(2,5-DiEt)Ph | |
| 957 | $-CH_2-O-C(=O)$ | -(2-Me)Ph | |
| 958 | – | -[1-(4-ClPhenyl)]cyclopropyl | |
| 959 | – | -[1-(3,4-DiClPhenyl)]cyclopropyl | |
| 960 | – | -[1-(4-Methoxyhenyl)]cyclopropyl | |
| 961 | – | (1-Methyl)cyclopropyl | |
| 962 | – | -[2,2-DiMethyl,3-(2,2'-DiBrom-Ethylen)]cyclopropyl | |
| 963 | $-CH_2$ | -H | |
| 964 | – | -Br | |
| 965 | $-CH_2$ | -4-(1-oxo)ethyl-Ph | |
| 966 | – | -4[1-(2-propenoxyimino)]Ethyl-Ph | |
| 967 | – | -4(1-Ethyloxyimino)Ethyl-Ph | |
| 968 | – | -4(1-Benzyloxyimino)Ethyl-Ph | |
| 969 | – | -4(2-propenoxyimino)Methyl-Ph | |
| 970 | – | -4(Ethyloxyimino)Methyl-Ph | |
| 971 | – | -4(Benzyloxyimino)Methyl-Ph | |

Tabelle 1 (Fortsetzung)

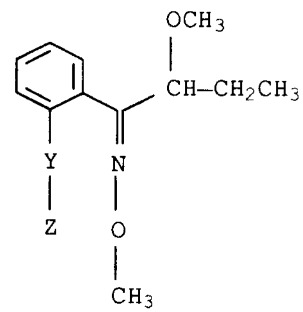

| Nr. | Y | Z | Fp. [°C] oder IR($cm^{-1}$) |
|---|---|---|---|
| 972 | $-CH_2O$ | -Ph | |
| 973 | - | -(2-Me)Ph | NMR: 7,70-6,70(m,8H); 5,05(m,2H); 3,85(m,4; 3,45(s,3H); 2,30(s,3H); 1,75-1,35(m,2H); 0,95(t,3H) ppm |
| 974 | - | -(2,4-DiMe)Ph | |
| 975 | - | -(4-Cl,2-Me)Ph | |
| 976 | - | -(4-Cl)Ph | |
| 977 | - | -(4-tBu,2 Me)Ph | |
| 978 | - | -(2,3,5-TriMe)Ph | |
| 979 | - | -(4-Cyclohexyl,2-Me)Ph | |
| 980 | - | -(4-Phenyl)Ph | |

| Nr. | Y | Z | Fp. [°C] oder IR($cm^{-1}$) |
|---|---|---|---|
| 981 | - | -(5-Me,2-iPr)Ph | |
| 982 | - | -(2,5-DiMe)Ph | |
| 983 | - | -(2,5-DiEt)Ph | |
| 984 | 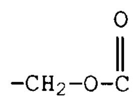 | -(2-Me)Ph | |
| 985 | - | -[1-(4-ClPhenyl)]cyclopropyl | |
| 986 | - | -[1-(3,4-DiClPhenyl)]cyclopropyl | |
| 987 | - | -[1-(4-Methoxyhenyl)]cyclopropyl | |
| 988 | - | (1-Methyl)cyclopropyl | |
| 989 | - | -[2,2-DiMethyl,3-(2,2'-DiBrom-Ethylen)]cyclopropyl | |
| 990 | $-CH_2$ | -H | |
| 991 | - | -Br | |
| 992 | $-CH_2$ | -4-(1-oxo)ethyl-Ph | |
| 993 | - | -4[1-(2-propenoxyimino)]Ethyl-Ph | |
| 994 | - | -4(1-Ethyloxyimino)Ethyl-Ph | |
| 995 | - | -4(1-Benzyloxyimino)Ethyl-Ph | |
| 996 | - | -4(2-propenoxyimino)Methyl-Ph | |
| 997 | - | -4(Ethyloxyimino)Methyl-Ph | |
| 998 | - | -4(Benzyloxyimino)Methyl-Ph | |

Tabelle 1 (Fortsetzung)

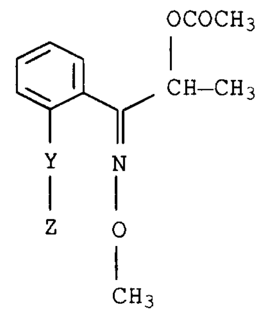

| Nr. | Y | Z | Fp. [°C] oder IR($cm^{-1}$) |
|---|---|---|---|
| 998 | $-CH_2O$ | -Ph | |
| 999 | - | -(2-Me)Ph | NMR: 7,70-6,70(m,8H); Isomer A 5,75(q,1H); Isomer B 5,65(q,1H); 4,95(m,2H); 3,85(m,3H); 2,30(s,3H); 2,00(m,3H); 1,45(m,3H) ppm |
| 1000 | - | -(2,4-DiMe)Ph | |
| 1001 | - | -(4-Cl,2-Me)Ph | |
| 1002 | - | -(4-Cl)Ph | |
| 1003 | - | -(4-tBu,2 Me)Ph | |
| 1004 | - | -(2,3,5-TriMe)Ph | |
| 1005 | - | -(4-Cyclohexyl,2-Me)Ph | |
| 1006 | - | -(4-Phenyl)Ph | |

| Nr. | Y | Z | Fp. [°C] oder IR($cm^{-1}$) |
|---|---|---|---|
| 1007 | - | -(5-Me,2-iPr)Ph | |
| 1008 | - | -(2,5-DiMe)Ph | |
| 1009 | - | -(2,5-DiEt)Ph | |
| 1010 | $-CH_2-O-\overset{O}{\overset{\|}{C}}$ | -(2-Me)Ph | |
| 1011 | - | -[1-(4-ClPhenyl)]cyclopropyl | |
| 1012 | - | -[1-(3,4-DiClPhenyl)]cyclopropyl | |
| 1013 | - | -[1-(4-Methoxyhenyl)]cyclopropyl | |
| 1014 | - | (1-Methyl)cyclopropyl | |
| 1015 | - | -[2,2-DiMethyl,3-(2,2'-DiBrom-Ethylen)]cyclopropyl | |
| 1016 | $-CH_2$ | -H | |
| 1017 | - | -Br | |
| 1018 | $-CH_2$ | -4-(1-oxo)ethyl-Ph | |
| 1019 | - | -4[1-(2-propenoxyimino)]Ethyl-Ph | |
| 1020 | - | -4(1-Ethyloxyimino)Ethyl-Ph | |
| 1021 | - | -4(1-Benzyloxyimino)Ethyl-Ph | |
| 1022 | - | -4(2-propenoxyimino)Methyl-Ph | |
| 1023 | - | -4(Ethyloxyimino)Methyl-Ph | |
| 1024 | - | -4(Benzyloxyimino)Methyl-Ph | |

Tabelle 1 (Fortsetzung)

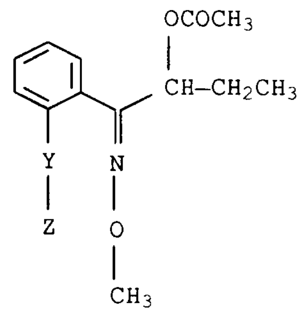

| Nr. | Y | Z | Fp. [°C] oder IR($cm^{-1}$) |
|---|---|---|---|
| 1025 | $-CH_2O$ | -Ph | |
| 1026 | - | -(2-Me)Ph | NMR: 7,70-6,75(m,8H); Isomer A 5,60(m,1H); Isomer B 5,56(m,1H); 4,95(m,2H); 3,85(m,2H); 2,30(s,3H); 2,10-1,65(m,5H); 0,95(t,3H) ppm |
| 1027 | - | -(2,4-DiMe)Ph | |
| 1028 | - | -(4-Cl,2-Me)Ph | |
| 1029 | - | -(4-Cl)Ph | |
| 1030 | - | -(4-tBu,2 Me)Ph | |
| 1031 | - | -(2,3,5-TriMe)Ph | |
| 1032 | - | -(4-Cyclohexyl,2-Me)Ph | |

| Nr. | Y | Z | Fp. [°C] oder IR($cm^{-1}$) |
|---|---|---|---|
| 1033 | - | -(4-Phenyl)Ph | |
| 1034 | - | -(5-Me,2-iPr)Ph | |
| 1035 | - | -(2,5-DiMe)Ph | |
| 1036 | - | -(2,5-DiEt)Ph | |
| 1037 | $-CH_2-O-\overset{O}{\overset{\|}{C}}$ | -(2-Me)Ph | |
| 1038 | - | -[1-(4-ClPhenyl)]cyclopropyl | |
| 1039 | - | -[1-(3,4-DiClPhenyl)]cyclopropyl | |
| 1040 | - | -[1-(4-Methoxyhenyl)]cyclopropyl | |
| 1041 | - | (1-Methyl)cyclopropyl | |
| 1042 | - | -[2,2-DiMethyl,3-(2,2'-DiBrom-Ethylen)]cyclopropyl | |
| 1043 | $-CH_2$ | -H | |
| 1044 | - | -Br | |
| 1045 | $-CH_2$ | -4-(1-oxo)ethyl-Ph | |
| 1046 | - | -4[1-(2-propenoxyimino)]Ethyl-Ph | |
| 1047 | - | -4(1-Ethyloxyimino)Ethyl-Ph | |
| 1048 | - | -4(1-Benzyloxyimino)Ethyl-Ph | |
| 1049 | - | -4(2-propenoxyimino)Methyl-Ph | |
| 1050 | - | -4(Ethyloxyimino)Methyl-Ph | |
| 1051 | - | -4(Benzyloxyimino)Methyl-Ph | |

Tabelle 1 (Fortsetzung)

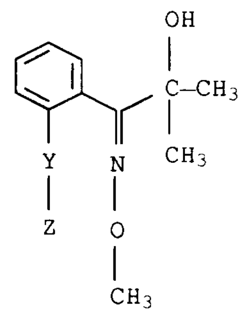

| Nr. | Y | Z | Fp. [°C] oder IR($cm^{-1}$) |
|---|---|---|---|
| 1052 | $-CH_2O$ | -Ph | |
| 1053 | - | -(2-Me)Ph | IR: 1495, 1242, 1230, 1066, 752 |
| 1054 | - | -(2,4-DiMe)Ph | |
| 1055 | - | -(4-Cl,2-Me)Ph | |
| 1056 | - | -(4-Cl)Ph | |
| 1057 | - | -(4-tBu,2 Me)Ph | |
| 1058 | - | -(2,3,5-TriMe)Ph | |
| 1059 | - | -(4-Cyclohexyl,2-Me)Ph | |
| 1060 | - | -(4-Phenyl)Ph | |
| 1061 | - | -(5-Me,2-iPr)Ph | |
| 1062 | - | -(2,5-DiMe)Ph | |

| Nr. | Y | Z | Fp. [°C] oder IR($cm^{-1}$) |
|---|---|---|---|
| 1063 | - | -(2,5-DiEt)Ph | |
| 1064 | $-CH_2-O-\overset{\overset{O}{\|}}{C}$ | -(2-Me)Ph | |
| 1065 | - | -[1-(4-ClPhenyl)]cyclopropyl | |
| 1066 | - | -[1-(3,4-DiClPhenyl)]cyclopropyl | |
| 1067 | - | -[1-(4-Methoxyhenyl)]cyclopropyl | |
| 1068 | - | (1-Methyl)cyclopropyl | |
| 1069 | - | -[2,2-DiMethyl,3-(2,2'-DiBrom-Ethylen)]cyclopropyl | |
| 1070 | $-CH_2$ | -H | |
| 1071 | - | -Br | |
| 1072 | $-CH_2$ | -4-(1-oxo)ethyl-Ph | |
| 1073 | - | -4[1-(2-propenoxyimino)]Ethyl-Ph | |
| 1074 | - | -4(1-Ethyloxyimino)Ethyl-Ph | |
| 1075 | - | -4(1-Benzyloxyimino)Ethyl-Ph | |
| 1076 | - | -4(2-propenoxyimino)Methyl-Ph | |
| 1077 | - | -4(Ethyloxyimino)Methyl-Ph | |
| 1078 | - | -4(Benzyloxyimino)Methyl-Ph | |

Tabelle 1 (Fortsetzung)

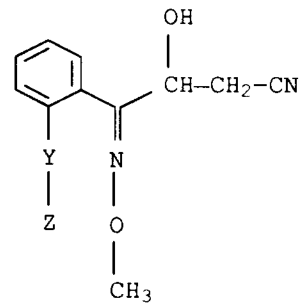

| Nr. | Y | Z | Fp. [°C] oder IR($cm^{-1}$) |
|---|---|---|---|
| 1079 | $-CH_2O$ | -Ph | |
| 1080 | - | -(2-Me)Ph | IR: 14,95, 1240, 1052, 1020, 753 |
| 1081 | - | -(2,4-DiMe)Ph | |
| 1082 | - | -(4-Cl,2-Me)Ph | |
| 1083 | - | -(4-Cl)Ph | |
| 1084 | - | -(4-tBu,2 Me)Ph | |
| 1085 | - | -(2,3,5-TriMe)Ph | |
| 1086 | - | -(4-Cyclohexyl,2-Me)Ph | |
| 1087 | - | -(4-Phenyl)Ph | |
| 1088 | - | -(5-Me,2-iPr)Ph | |
| 1089 | - | -(2,5-DiMe)Ph | |

| Nr. | Y | Z | Fp. [°C] oder IR($cm^{-1}$) |
|---|---|---|---|
| 1090 | – | -(2,5-DiEt)Ph | |
| 1091 | $-CH_2-O-C(=O)$ | -(2-Me)Ph | |
| 1092 | – | -[1-(4-ClPhenyl)]cyclopropyl | |
| 1093 | – | -[1-(3,4-DiClPhenyl)]cyclopropyl | |
| 1094 | – | -[1-(4-Methoxyhenyl)]cyclopropyl | |
| 1095 | – | (1-Methyl)cyclopropyl | |
| 1096 | – | -[2,2-DiMethyl,3-(2,2'-DiBrom-Ethylen)]cyclopropyl | |
| 1097 | $-CH_2$ | -H | |
| 1098 | – | -Br | |
| 1099 | $-CH_2$ | -4-(1-oxo)ethyl-Ph | |
| 1100 | – | -4[1-(2-propenoxyimino)]Ethyl-Ph | |
| 1101 | – | -4(1-Ethyloxyimino)Ethyl-Ph | |
| 1102 | – | -4(1-Benzyloxyimino)Ethyl-Ph | |
| 1103 | – | -4(2-propenoxyimino)Methyl-Ph | |
| 1104 | – | -4(Ethyloxyimino)Methyl-Ph | |
| 1105 | – | -4(Benzyloxyimino)Methyl-Ph | |

Tabelle 1 (Fortsetzung)

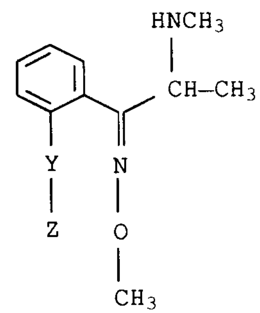

| Nr. | Y | Z | Fp. [°C] oder IR($cm^{-1}$) |
|---|---|---|---|
| 1106 | $-CH_2O$ | -Ph | |
| 1107 | - | -(2-Me)Ph | NMR: 7,70-6,75(m,8H); 4,95(m,2H); 3,85(s,3H); 3,50(m,1H), 2,50(m,3H); 2,30(s,3H); 1,1(m,3H) ppm |
| 1108 | - | -(2,4-DiMe)Ph | |
| 1109 | - | -(4-Cl,2-Me)Ph | |
| 1110 | - | -(4-Cl)Ph | |
| 1111 | - | -(4-tBu,2 Me)Ph | |
| 1112 | - | -(2,3,5-TriMe)Ph | |
| 1113 | - | -(4-Cyclohexyl,2-Me)Ph | |
| 1114 | - | -(4-Phenyl)Ph | |

| Nr. | Y | Z | Fp. [°C] oder IR($cm^{-1}$) |
|---|---|---|---|
| 1115 | - | -(5-Me,2-iPr)Ph | |
| 1116 | - | -(2,5-DiMe)Ph | |
| 1117 | - | -(2,5-DiEt)Ph | |
| 1118 | O<br>‖<br>$-CH_2-O-C$ | -(2-Me)Ph | |
| 1119 | - | -[1-(4-ClPhenyl)]cyclopropyl | |
| 1120 | - | -[1-(3,4-DiClPhenyl)]cyclopropyl | |
| 1121 | - | -[1-(4-Methoxyhenyl)]cyclopropyl | |
| 1122 | - | (1-Methyl)cyclopropyl | |
| 1123 | - | -[2,2-DiMethyl,3-(2,2'-DiBrom-Ethylen)]cyclopropyl | |
| 1124 | $-CH_2$ | -H | |
| 1125 | - | -Br | |
| 1126 | $-CH_2$ | -4-(1-oxo)ethyl-Ph | |
| 1127 | - | -4[1-(2-propenoxyimino)]Ethyl-Ph | |
| 1128 | - | -4(1-Ethyloxyimino)Ethyl-Ph | |
| 1129 | - | -4(1-Benzyloxyimino)Ethyl-Ph | |
| 1130 | - | -4(2-propenoxyimino)Methyl-Ph | |
| 1131 | - | -4(Ethyloxyimino)Methyl-Ph | |
| 1132 | - | -4(Benzyloxyimino)Methyl-Ph | |

Tabelle 1 (Fortsetzung)

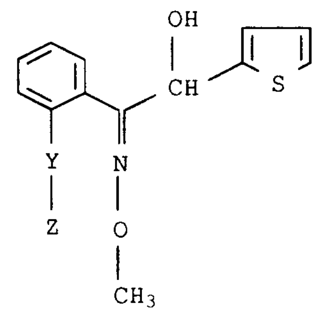

| Nr. | Y | Z | Fp. [°C] oder IR($cm^{-1}$) |
|---|---|---|---|
| 1160 | $-CH_2O$ | -Ph | |
| 1161 | - | -(2-Me)Ph | IR: 1494, 1243, 1229, 1021, 751 |
| 1162 | - | -(2,4-DiMe)Ph | |
| 1163 | - | -(4-Cl,2-Me)Ph | |
| 1164 | - | -(4-Cl)Ph | |
| 1165 | - | -(4-tBu,2 Me)Ph | |
| 1166 | - | -(2,3,5-TriMe)Ph | |
| 1167 | - | -(4-Cyclohexyl,2-Me)Ph | |
| 1168 | - | -(4-Phenyl)Ph | |
| 1169 | - | -(5-Me,2-iPr)Ph | |
| 1170 | - | -(2,5-DiMe)Ph | |

| Nr. | Y | Z | Fp. [°C] oder IR($cm^{-1}$) |
|---|---|---|---|
| 1171 | - | -(2,5-DiEt)Ph | |
| 1172 | 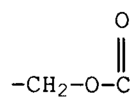 | -(2-Me)Ph | |
| 1173 | - | -[1-(4-ClPhenyl)]cyclopropyl | |
| 1174 | - | -[1-(3,4-DiClPhenyl)]cyclopropyl | |
| 1175 | - | -[1-(4-Methoxyhenyl)]cyclopropyl | |
| 1176 | - | (1-Methyl)cyclopropyl | |
| 1177 | - | -[2,2-DiMethyl,3-(2,2'-DiBrom-Ethylen)]cyclopropyl | |
| 1178 | $-CH_2$ | -H | |
| 1179 | - | -Br | |
| 1180 | $-CH_2$ | -4-(1-oxo)ethyl-Ph | |
| 1181 | - | -4[1-(2-propenoxyimino)]Ethyl-Ph | |
| 1182 | - | -4(1-Ethyloxyimino)Ethyl-Ph | |
| 1183 | - | -4(1-Benzyloxyimino)Ethyl-Ph | |
| 1184 | - | -4(2-propenoxyimino)Methyl-Ph | |
| 1185 | - | -4(Ethyloxyimino)Methyl-Ph | |
| 1186 | - | -4(Benzyloxyimino)Methyl-Ph | |

Tabelle 1 (Fortsetzung)

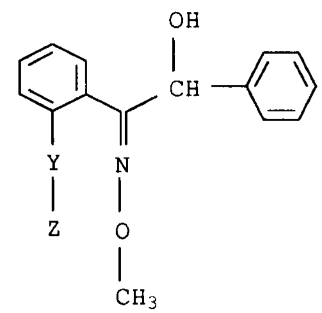

| Nr. | Y | Z | Fp. [°C] oder IR($cm^{-1}$) |
|---|---|---|---|
| 1187 | $-CH_2O$ | -Ph | |
| 1188 | - | -(2-Me)Ph | NMR: 7,70-6,70(m,8H); 4,95(m,2H); 4,45(m,1H); 3,90(s,3H); 2,30(s,3H); 1,60(m,2H); 1,00(t,3H) ppm |
| 1189 | - | -(2,4-DiMe)Ph | |
| 1190 | - | -(4-Cl,2-Me)Ph | |
| 1191 | - | -(4-Cl)Ph | |
| 1192 | - | -(4-tBu,2 Me)Ph | |
| 1193 | - | -(2,3,5-TriMe)Ph | |
| 1194 | - | -(4-Cyclohexyl,2-Me)Ph | |
| 1195 | - | -(4-Phenyl)Ph | |

| Nr. | Y | Z | Fp. [°C] oder IR($cm^{-1}$) |
|---|---|---|---|
| 1196 | - | -(5-Me,2-iPr)Ph | |
| 1197 | - | -(2,5-DiMe)Ph | |
| 1198 | - | -(2,5-DiEt)Ph | |
| 1199 | $-CH_2-O-C(=O)$ | -(2-Me)Ph | |
| 1200 | - | -[1-(4-ClPhenyl)]cyclopropyl | |
| 1201 | - | -[1-(3,4-DiClPhenyl)]cyclopropyl | |
| 1202 | - | -[1-(4-Methoxyhenyl)]cyclopropyl | |
| 1203 | - | (1-Methyl)cyclopropyl | |
| 1204 | - | -[2,2-DiMethyl,3-(2,2'-DiBrom-Ethylen)]cyclopropyl | |
| 1205 | $-CH_2$ | -H | |
| 1206 | - | -Br | |
| 1207 | $-CH_2$ | -4-(1-oxo)ethyl-Ph | |
| 1208 | - | -4[1-(2-propenoxyimino)]Ethyl-Ph | |
| 1209 | - | -4(1-Ethyloxyimino)Ethyl-Ph | |
| 1210 | - | -4(1-Benzyloxyimino)Ethyl-Ph | |
| 1211 | - | -4(2-propenoxyimino)Methyl-Ph | |
| 1212 | - | -4(Ethyloxyimino)Methyl-Ph | |
| 1213 | - | -4(Benzyloxyimino)Methyl-Ph | |

Tabelle 1 (Fortsetzung)

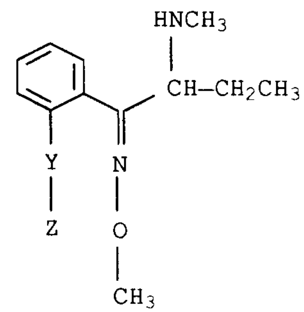

| Nr. | Y | Z | Fp. [°C] oder IR($cm^{-1}$) |
|---|---|---|---|
| 1216 | $-CH_2O$ | -Ph | |
| 1217 | - | -(2-Me)Ph | IR: 1495, 1241, 1229, 1013, 750 |
| 1218 | - | -(2,4-DiMe)Ph | |
| 1219 | - | -(4-Cl,2-Me)Ph | |
| 1220 | - | -(4-Cl)Ph | |
| 1221 | - | -(4-tBu,2 Me)Ph | |
| 1222 | - | -(2,3,5-TriMe)Ph | |
| 1223 | - | -(4-Cyclohexyl,2-Me)Ph | |
| 1224 | - | -(4-Phenyl)Ph | |
| 1225 | - | -(5-Me,2-iPr)Ph | |
| 1226 | - | -(2,5-DiMe)Ph | |

| Nr. | Y | Z | Fp. [°C] oder IR($cm^{-1}$) |
|---|---|---|---|
| 1227 | - | -(2,5-DiEt)Ph | |
| 1228 | $-CH_2-O-\overset{O}{\overset{\|}{C}}$ | -(2-Me)Ph | |
| 1229 | - | -[1-(4-ClPhenyl)]cyclopropyl | |
| 1230 | - | -[1-(3,4-DiClPhenyl)]cyclopropyl | |
| 1231 | - | -[1-(4-Methoxyhenyl)]cyclopropyl | |
| 1232 | - | (1-Methyl)cyclopropyl | |
| 1233 | - | -[2,2-DiMethyl,3-(2,2'-DiBrom-Ethylen)]cyclopropyl | |
| 1234 | $-CH_2$ | -H | |
| 1235 | - | -Br | |
| 1236 | $-CH_2$ | -4-(1-oxo)ethyl-Ph | |
| 1237 | - | -4[1-(2-propenoxyimino)]Ethyl-Ph | |
| 1238 | - | -4(1-Ethyloxyimino)Ethyl-Ph | |
| 1239 | - | -4(1-Benzyloxyimino)Ethyl-Ph | |
| 1240 | - | -4(2-propenoxyimino)Methyl-Ph | |
| 1241 | - | -4(Ethyloxyimino)Methyl-Ph | |
| 1242 | - | -4(Benzyloxyimino)Methyl-Ph | |

Tabelle 1 (Fortsetzung)

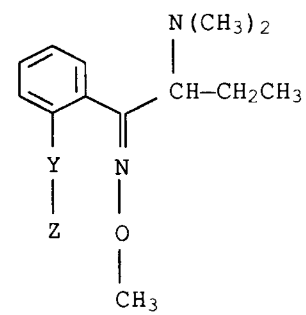

| Nr. | Y | Z | Fp. [°C] oder IR($cm^{-1}$) |
|---|---|---|---|
| 1243 | $-CH_2O$ | -Ph | |
| 1244 | - | -(2-Me)Ph | IR: 1495, 1241, 1228, 1015, 750 |
| 1245 | - | -(2,4-DiMe)Ph | |
| 1246 | - | -(4-Cl,2-Me)Ph | |
| 1247 | - | -(4-Cl)Ph | |
| 1248 | - | -(4-tBu,2 Me)Ph | |
| 1249 | - | -(2,3,5-TriMe)Ph | |
| 1250 | - | -(4-Cyclohexyl,2-Me)Ph | |
| 1251 | - | -(4-Phenyl)Ph | |
| 1252 | - | -(5-Me,2-iPr)Ph | |
| 1253 | - | -(2,5-DiMe)Ph | |
| 1254 | - | -(2,5-DiEt)Ph | |

| Nr. | Y | Z | Fp. [°C] oder IR($cm^{-1}$) |
|---|---|---|---|
| 1255 | $-CH_2-O-\overset{\overset{O}{\|}}{C}$ | -(2-Me)Ph | |
| 1256 | - | -[1-(4-ClPhenyl)]cyclopropyl | |
| 1257 | - | -[1-(3,4-DiClPhenyl)]cyclopropyl | |
| 1258 | - | -[1-(4-Methoxyhenyl)]cyclopropyl | |
| 1259 | - | (1-Methyl)cyclopropyl | |
| 1260 | - | -[2,2-DiMethyl,3-(2,2'-DiBrom-Ethylen)]cyclopropyl | |
| 1261 | $-CH_2$ | -H | |
| 1262 | - | -Br | |
| 1263 | $-CH_2$ | -4-(1-oxo)ethyl-Ph | |
| 1264 | - | -4[1-(2-propenoxyimino)]Ethyl-Ph | |
| 1265 | - | -4(1-Ethyloxyimino)Ethyl-Ph | |
| 1266 | - | -4(1-Benzyloxyimino)Ethyl-Ph | |
| 1267 | - | -4(2-propenoxyimino)Methyl-Ph | |
| 1268 | - | -4(Ethyloxyimino)Methyl-Ph | |
| 1269 | - | -4(Benzyloxyimino)Methyl-Ph | |

Die neuen Oximether eignen sich als Fungizide.

Die erfindungsgemäßen fungiziden Oximether bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet

werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon, Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Oximether zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Oximether zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Unicinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosphorium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,

Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Anwendungsbeispiel

Als Vergleichswirkstoff wurde 1-Methoxyimino-1-(2-phenoxymethyl)-phenylessigsäuremethylester (A) - bekannt aus EP 253 213 - benutzt.

Anwendungsbeispiel

Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff 918 bei der Anwendung als 0,0015 %ige (Gew.-%) Spritzbrühe eine bessere fungizide Wirkung zeigen (95 %) als der bekannte Vergleichswirkstoff A (75 %).

**Patentansprüche**

1. Oximether der Formel I

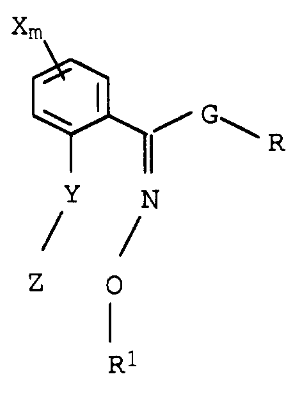

I,

in der
G

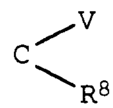

bedeutet,

V $O-R^6$,

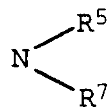

bedeutet,

R gegebenenfalls substituiertes $C_3$-$C_8$-Cycloalkyl, gegebenenfalls substituiertes $C_2$-$C_8$-Alkenyl, gegebenenfalls substituiertes $C_2$-$C_8$-Alkinyl, gegebenenfalls substituiertes Phenyl, gegebenenfalls substituiertes Hetaryl oder

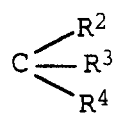

bedeutet,

$R^1$ Wasserstoff, $C_1$-$C_8$-Alkyl bedeutet,

$R^2$ Wasserstoff, $C_1$-$C_8$-Alkyl, Aralkyl, Hetarylalkyl, Alkoxyalkyl, Aryloxyalkyl, Aryl, Hetaryl bedeutet,

$R^3$ Wasserstoff, Methyl, -COO-A bedeutet,

A Wasserstoff, $C_1$-$C_8$-Alkyl bedeutet, oder für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$ Wasserstoff, $C_1$-$C_8$-Alkyl, Aralkyl, Hetarylalkyl, Alkoxyalkyl, Aryloxyalkyl, Aryl, Hetaryl bedeutet, oder

$R^2$ + $R^4$ zusammen mit dem C, dessen Substituenten sie sind, einen $C_3$-$C_8$-Cycloalkylring bedeuten,

$R^5$ Wasserstoff, $C_1$-$C_8$-Alkyl, $C_1$-$C_4$-Aralkyl, $C_3$-$C_6$-Cycloalkyl, Hydroxy, $C_1$-$C_4$-Alkyloxy, $NH_2$, $C_1$-$C_4$-Alkylamino, $C_1$-$C_4$-Dialkylamino bedeutet,

$R^6$ Wasserstoff, $C_1$-$C_8$-Alkyl, $C_1$-$C_4$-Alkanoyl bedeutet,

$R^7$ Wasserstoff, $C_1$-$C_8$-Alkyl, $C_1$-$C_4$-Alkanoyl bedeutet,

$R^8$ Wasserstoff, $C_1$-$C_8$-Alkyl bedeutet,

Y gegebenenfalls substituiertes $C_1$-$C_8$-Alkyl, gegebenenfalls substituiertes $C_2$-$C_8$-Alkenyl, $C_2$-$C_8$-Alkinyl, O, $S(O)_m$ (m = 0, 1, 2), N, das substituiert ist durch gegebenenfalls substituiertes $C_1$-$C_8$-Alkyl, Oxycarbonyl, Carbonyloxy, Oxycarbonyl-$C_1$-$C_8$-alkyl, Carbonyloxy-$C_1$-$C_8$-alkyl, $C_2$-$C_8$-Oxyalkyloxy, $C_2$-$C_8$-Orvalkenyl, $C_1$-$C_8$-Alkyloxy, Oxy-$C_1$-$C_4$-alkyl, $C_1$-$C_8$-Thioalkyl, Azo, gegebenenfalls $C_1$-$C_8$-Alkylsubstituiertes Carbonylamino, gegebenenfalls $C_1$-$C_8$-alkylsubstituiertes Aminocarbonyl, gegebenenfalls $c_1$-$C_8$-alkylsubstituiertes Aminocarbonyloxy, Oxyimino, gegebenenfalls substituiertes Iminooxyalkyl bedeutet,

Z $C_1$-$C_{18}$-Alkyl, $C_2$-$C_{18}$-Alkenyl, $C_3$-$C_8$-Alkinyl, Aryl, Aryl-$C_1$-$C_{10}$-Alkyl, Aryl-$C_2$-$C_{10}$-Alkenyl, Aryloxy-$C_1$-$C_{10}$-Alkyl, $C_1$-$C_4$-Alkoxy-$C_1$-$C_{10}$-alkyl, $C_1$-$C_{10}$-Halogenalkyl, $C_1$-$C_4$-Alkoxycarbonyl, Hetaryl bedeutet,

Z bedeutet für den Fall, daß Y nicht Oxyalkyl, Oxyalkyloxy, Thioalkyl, -O-N=C-, -N=N, Oxycarbonyl, Oxycarbonyloxy, O oder S ist, zusätzlich noch Aryloxy, Aryloxy-$C_1$-$C_{10}$-alkoxy, Aryl-$C_1$-$C_{10}$-alkoxy, $C_1$-$C_{10}$-Alkoxy oder Hetaryloxy,

Z bedeutet für den Fall, daß Y nicht Oxyalkyl, Oxyalkyloxy, -O-N=C, -N=N-, Oxycarbonyl, Oxycarbonylalkyl oder O ist, zusätzlich noch $C_1$-$C_{18}$-Alkylthio oder Arylt-

hio,

wobei die für Z genannten Reste gegebenenfalls substituiert sind durch Halogen, Cyano, Nitro, $C_1$-$C_4$-Alkyl, $C_2$-$C_4$-Alkenyl, $C_1$-$C_4$-Alkoxy, $C_1$-$C_4$-Halogenalkyl, $C_1$-$C_4$-Alkoxy-$C_1$-$C_4$-alkyl, $C_1$-$C_4$--dialkoxy-$C_1$-$C_4$-Alkyl, gegebenenfalls substituiertes O-$C_1$-$C_{10}$-Alkyloxyimino, gegebenenfalls substituiertes $C_2$-$C_{10}$-Alkanoyl, gegebenenfalls substituiertes Formyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten einen gegebenenfalls substituierten aromatischen oder heteroaromatischen Ring bilden können,

X gleich oder verschieden ist und Wasserstoff, Halogen, Nitro, gegebenenfalls substituiertes $C_1$-$C_4$-Alkyl, gegebenenfalls substituiertes $C_1$-$C_4$-Alkoxy oder gegebenenfalls substituiertes $C_1$-$C_4$-Halogenalkyl bedeutet und

m eine ganze Zahl zwischen 1 und 4 bedeutet.

**2.** Oximether der Formel I gemäß Anspruch 1, in der der G CH-OH, R $CR^2$, $R^3$, $R^4$, $R^1$ Methyl, $R^2$ und $R^3$ Wasserstoff, $R^4$ Methyl, Y Oxymethylen, Z 2-Methylphenyl bedeutet.

**3.** Verfahren zur Bekämpfung von Pilzen, dadurch gekennzeichnet, daß man die Pilze oder die von Pilzbefall bedrohten Materialien, Pflanzen, Saatgüter oder den Erdboden mit eine fungizid wirksamen Menge einer Verbindung der Formel I gemäß Anspruch 1 behandelt.

**4.** Fungizid, enthaltend einen inerten Trägerstoff und ein fungizid wirksame Menge einer Verbindung der Formel I gemäß Anspruch 1.

**5.** Oximether der Formel I

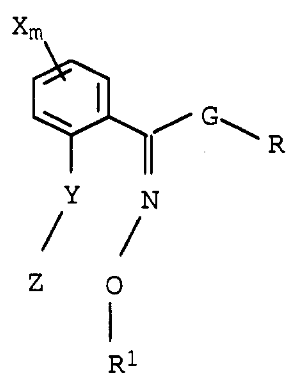

I,

in der

G

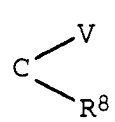

bedeutet,

V O-$R^6$,

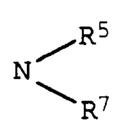

bedeutet,

R gegebenenfalls substituiertes $C_3$-$C_8$-Cycloalkyl, gegebenenfalls substituiertes $C_2$-$C_8$-Alkenyl, gegebenenfalls substituiertes $C_2$-$C_8$-Alkinyl, gegebenenfalls substituiertes Phenyl, gegebenenfalls substituiertes Hetaryl oder

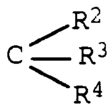

bedeutet,

$R^1$ Wasserstoff, $C_1$-$C_8$-Alkyl bedeutet,

$R^2$ Wasserstoff, $C_1$-$C_8$-Alkyl, Aralkyl, Hetarylalkyl, Alkoxyalkyl, Aryloxyalkyl, Aryl, Hetaryl bedeutet,

$R^3$ Wasserstoff, Methyl, -COO-A bedeutet,

A Wasserstoff, $C_1$-$C_8$-Alkyl bedeutet, oder für den Fall, daß $R^3$ den Rest -COO-$C_1$-$C_8$-Alkyl bedeutet, $R^2$ und $R^3$ zusammen mit dem C, dessen Substituenten sie sind, einen 5 bis 8-gliedrigen Lactonring bedeuten,

$R^4$ Wasserstoff, $C_1$-$C_8$-Alkyl, Aralkyl, Hetarylalkyl, Alkoxyalkyl, Aryloxyalkyl, Aryl, Hetaryl bedeutet, oder

$R^2$ + $R^4$ zusammen mit dem C, dessen Substituenten sie sind, einen $C_3$-$C_8$-Cycloalkylring bedeuten,

$R^5$ Wasserstoff, $C_1$-$C_8$-Alkyl, $C_1$-$C_4$-Aralkyl, $C_3$-$C_6$-Cycloalkyl, Hydroxy, $C_1$-$C_4$-Alkyloxy, $NH_2$, $C_1$-$C_4$-Alkylamino, $C_1$-$C_4$-Dialkylamino bedeutet,

$R^6$ Wasserstoff, $C_1$-$C_8$-Alkyl, $C_1$-$C_4$-Alkanoyl bedeutet,

$R^7$ Wasserstoff, $C_1$-$C_8$-Alkyl, $C_1$-$C_4$-Alkanoyl bedeutet,

$R^8$ Wasserstoff, $C_1$-$C_8$-Alkyl bedeutet,

Y gegebenenfalls substituiertes $C_1$-$C_8$-Alkyl, gegebenenfalls substituiertes $C_2$-$C_8$-Alkenyl, $C_2$-$C_8$-Alkinyl, O, $S(O)_m$ (m = 0, 1, 2), N, das substituiert ist durch gegebenenfalls substituiertes $C_1$-$C_8$-Alkyl, Oxycarbonyl, Carbonyloxy, Oxycarbonyl-$C_1$-$C_8$-alkyl, Carbonyloxy-$C_1$-$C_8$-alkyl, $C_2$-$C_8$-Oxyalkyloxy, $C_2$-$C_8$-Oxyalkenyl, $C_1$-$C_8$-Alkyloxy, Oxy-$C_1$-$C_4$-alkyl, $C_1$-$C_8$-Thioalkyl, Azo, gegebenenfalls $C_1$-$C_8$-Alkylsubstituiertes Carbonylamino, gegebenenfalls $C_1$-$C_8$-alkylsubstituiertes Aminocarbonyl, gegebenenfalls $c_1$-$C_8$-alkylsubstituiertes Aminocarbonyloxy, Oxyimino, gegebenenfalls substituiertes Iminooxyalkyl bedeutet,

Z $C_1$-$C_{18}$-Alkyl, $C_2$-$C_{18}$-Alkenyl, $C_3$-$C_8$-Alkinyl, Aryl, Aryl-$C_1$-$C_{10}$-Alkyl, Aryl-$C_2$-$C_{10}$-Alkenyl, Aryloxy-$C_1$-$C_{10}$-Alkyl, $C_1$-$C_4$-Alkoxy-$C_1$-$C_{10}$-alkyl, $C_1$-$C_{10}$-Halogenalkyl, $C_1$-$C_4$-Alkoxycarbonyl, Hetaryl bedeutet,

Z bedeutet für den Fall, daß Y nicht Oxyalkyl, Oxyalkyloxy, Thioalkyl, -O-N=C-, -N=N, Oxycarbonyl, Oxycarbonyloxy, O oder S ist, zusätzlich noch Aryloxy, Aryloxy-$C_1$-$C_{10}$-alkoxy, Aryl-$C_1$-$C_{10}$-alkoxy, $C_1$-$C_{10}$-Alkoxy oder Hetaryloxy,

Z bedeutet für den Fall, daß Y nicht Oxyalkyl, Oxyalkyloxy, -O-N=C, -N=N-, Oxycarbonyl, Oxycarbonylalkyl oder O ist, zusätzlich noch $C_1$-$C_{18}$-Alkylthio oder Arylthio,

wobei die für Z genannten Reste gegebenenfalls substituiert sind durch Halogen, Cyano, Nitro, $C_1$-$C_4$-Alkyl, $C_2$-$C_4$-Alkenyl, $C_1$-$C_4$-Alkoxy, $C_1$-$C_4$-Halogenalkyl, $C_1$-$C_4$-Alkoxy-$C_1$-$C_4$-alkyl, $C_1$-$C_4$--dialkoxy-$C_1$-$C_4$-Alkyl, gegebenenfalls substituiertes O-$C_1$-$C_{10}$-Alkyloxyimino, gegebenenfalls substituiertes $C_2$-$C_{10}$-Alkanoyl, gegebenenfalls substituiertes Formyl, $C_2$-$C_4$-Halogenalkenyl, $C_1$-$C_4$-Alkoxycarbonyl, gegebenenfalls substituiertes Phenyl, gegebenenfalls substituiertes Phenoxy bedeutet, oder einen C-verknüpften, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten einen gegebenenfalls substituierten aromatischen oder heteroaromatischen Ring bilden können,

X gleich oder verschieden ist und Wasserstoff, Halogen, Nitro, gegebenenfalls substituiertes $C_1$-$C_4$-Alkyl, gegebenenfalls substituiertes $C_1$-$C_4$-Alkoxy oder gegebenenfalls substituiertes $C_1$-$C_4$-Halogenalkyl bedeutet und

m eine ganze Zahl zwischen 1 und 4 bedeutet.

Europäisches Patentamt

# EUROPÄISCHER RECHERCHENBERICHT

Nummer der Anmeldung
EP 95 11 0459

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 006 254 (SHELL INTERNATIONALE RESEARCH MAATSCHAPPIJ)<br>* Ansprüche; Beispiele *<br>--- | 1-5 | C07C251/48<br>C07C255/64<br>C07D333/22<br>A01N35/10<br>A01N37/50<br>A01N43/10 |
| A | EP-A-0 254 426 (IMPERIAL CHEMICAL INDUSTRIES)<br>* Ansprüche; Tabelle I *<br>--- | 1-5 | |
| A | EP-A-0 407 891 (BASF AG)<br>* Ansprüche; Beispiel 1; Tabelle 1 *<br>--- | 1-5 | |
| A | EP-A-0 363 818 (BASF AG)<br>* Ansprüche; Tabelle 1 *<br>----- | 1-5 | |

RECHERCHIERTE SACHGEBIETE (Int.Cl.5)

C07C
A01N
C07D

Der vorliegende Recherchenbericht wurde für alle Patentansprüche erstellt

| Recherchenort | Abschlußdatum der Recherche | Prüfer |
|---|---|---|
| DEN HAAG | 22. August 1995 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)